# EUROPEAN PATENT SPECIFICATION

(11) **EP 0 756 630 B1**
(45) Date of publication and mention of the grant of the patent: **23.07.2003**
(21) Application number: 95915295.0
(22) Date of filing: 18.04.1995
(51) Int. Cl.: C12N 15/31, C07K 14/195

(54) **RECOMBINANT PGP3, METHODS OF PREPARATION AND USE IN DIAGNOSIS AND THERAPY**
REKOMBINANT PGP3, VERFAHREN ZU DESSEN HERSTELLUNG, UND DESSEN VERWENDUNG IN DER DIAGNOSE UND THERAPIE
PROTEINE A CODAGE PLASMIDIQUE DE RECOMBINAISON PGP3, METHODES DE PREPARATION ET D'UTILISATION EN DIAGNOSTIC ET EN THERAPIE

(30) Priority: 19.04.1994 US 229980
(43) Date of publication of application: 05.02.1997
(73) Proprietor: Chiron S.p.A., 53100 Siena (IT)
(72) Inventor: RATTI, Giulio, I-53100 Siena (IT)
(74) Representative: Hallybone, Huw George
(86) International application number: PCT/IB95/00310
(87) International publication number: WO 95/028487

(56) References cited:
- EP-A- 0 336 412
- EP-A- 0 499 681
- J. GEN. MICROBIOL., vol. 139, 1993 pages 1083-1092, COMANDUCCI M. ET AL. 'Expression of a plasmid gene of Chlamydia trachomatis encoding a novel 28kDa antigen' cited in the application
- INFECTION AND IMMUNITY, vol. 62, no. 12, December 1994 pages 5491-5497, COMANDUCCI M. ET AL. 'Humoral immune response to plasmid protein pgp3 in patients with chlamydia trachomatis infection'
- PLASMID, vol. 18, 1987 pages 205-214, SRIPRAKASH K.S. AND MACAVOY E.S. 'Characterisation and sequence of a plasmid from the Trachoma biovar of Clamydia trachomatis' cited in the application

## Description

### Field of the Invention

The invention relates to a recombinant form of plasmid-encoded protein pgp3 from *Chlamydia trachomatis*, serotype D and the uses thereof in immunoassay, particularly quantitative immunoassay, such as enzyme linked immunoassay (ELISA). The invention also relates to methods of production of pgp3.

### Background to the Invention

Chlamydias are gram-negative bacteria which are obligate intracellular parasites of eukaryotic cells. They exhibit a form which is extracellular, infective and metabolically practically inert, known as the elemental body (EB) and an intracellular replicative form called the reticular body (RB).

The reticular bodies, after multiplication by binary fission, are transformed into elemental bodies which are released from the host cell and infect new cells. The masses of reticular and elemental bodies inside an infected cell constitute characteristic "inclusions" visible with an optical microscope.

*Chlamydia trachomatis* (*C. trachomatis* or CT) is a bacterial species pathogenic to man and is the etiological agent of venereal lymphogranuloma (VLG) which gives rise to various inflammatory pathologies of the human genitalia and of trachoma, a chronic disease which affects 500 million people and can cause blindness. Urethritis and cervicitis induced by *C. trachomatis* if not treated early can lead to chronic inflammations such as vaginitis, salpingitis and pelvic inflammation which may result in sterility and extrauterine pregnancy. Furthermore, the newborn from infected mothers may contract pulmonary and/or ocular infections during delivery.

There is therefore a clear and unfulfilled need for accurate diagnostic assay techniques for detecting *C. trachomatis* infection as well immunological preparations for use in the prevention of infection and the treatment of preexisting infection.

*C. trachomatis* (15,16) harbours a conserved 7.5-kb plasmid (pCT) (3; see also EP-A-0 499 681), which appears to be under positive selective pressure during natural infections, since it is found in essentially all strains and isolates. The role that this genetic element may have in *C.trachomatis* physiology has been object of speculation; however, the search for pCT-associated phenotypes has been hampered by the fact that no genetic transformation procedure for chlamydia has been available, so far.

The identification and characterization of plasmid-encoded products is of particular interest, because pCT could encode chlamydial pathogenicity factors, as is the case for plasmids of several other pathogenic bacteria. Although the origin of replication has been identified (28), and several features of pCT transcriptional regulation are known (20, 21, 5), the majority of other pCT features are only hypotheses deduced from DNA sequencing data (2, 3, 8, 27). However, a 28-kDa electrophoretic band has been recently reported to cross-react on Western blots of chlamydial protein extracts with antibodies raised against a 39 kDa chimeric protein, obtained by gene fusion with pCT open reading frame 3 (ORF3) (4). We have conclusively identified a 28-kDa component of *C.trachomatis* elementary bodies (EBs) as the plasmid-encoded polypeptide pgp3. Also, using an immunoassay with a purified, recombinant form of this protein, we show that it represents a novel and potentially important immunogen in human chlamydial infections.

Initial attempts to develop an ELISA test for human sera using the previously described (4) 39-kDa pgp3 fusion protein, purified by electrophoresis on SDS-acrylamide gels, gave inconsistent and unsatisfactory results. One of the problems was the observation that, in some patient sera, antibody reaction with minor contaminants from *E. coli* was stronger than the reaction with the denatured fusion protein.

Situations like this can be adequately resolved by using the Western blot technique, where signals from different antigens can be separately monitored, but they become a serious problem in ELISA. For example, the resulting 39-kDa product was used to show that pgp3 epitopes can be recognised on Western blots by antibodies present in sera from patients with chlamydial infections, but not in control sera from healthy donors.

The object of the present invention is to provide a serological test system more suitable than the immunoblotting technique for obtaining reproducible and quantitative data from large numbers of clinical samples.

We attempted to improve both the quality of the recombinant antigen and purification procedures to produce a reagent suitable for use in ELISA.

The present invention therefore relates to an enzyme-linked immunoassay based on a new recombinant form of the pgp3 protein which can be used for assessing the prevalence of pgp3 antibodies in people with *C.trachomatis* infections.

r-pgp3 was obtained as stable 28-kDa protein, which remained water-soluble in the bacterial cell even when over-expressed by the pT7-7 system, and throughout the whole purification procedure. Since mis-folded recombinant proteins typically undergo proteolytic degradation, or tend to aggregate and precipitate (34), the above properties suggest that r-pgp3 (which contains four cysteine residues) is correctly folded in a structure close to its native form. We believe that r-pgp3 may also retain an antigenic structure capable of detecting antibodies directed against conformational epitopes, which could be missed by immunoblot analysis.

Surprisingly, a high proportion of r-pgp3 was found in the periplasm of the transformed *E.coli* BL21 cells, and the possibility of using periplasmic extracts simplified its purification.

### Summary of the Invention

It has been established that the conformation of pgp3 is important for the retention of essential immunological properties. According to a first aspect of the invention, there is provided pgp3 protein as defined in claims 1 to 3.

The recombinant pgp3 protein may be a recombinant form of any of the allelic variants of the pgp3 protein having a molecular weight of about 28kd, including for example pgp3-D (the allele from *C.trachomatis* serotype D) or pgp3-L2 (the allele from *C.trachomatis* L2. pgp3-D is a prototype of the trachoma variants of *C.trachomatis* and pgp3-L2 is a prototype of the lymphogranuloma variants of *C.trachomatis*.

Preferably the recombinant protein of the first aspect of the invention is substantially in a conformation capable of recognition by antibodies in human serum. This protein is a new recombinant form of pgp3, not previously described in the literature which has advantageous properties when used in immunoassay, particularly enzyme-linked immunoassays.

The protein may be a derivative of pgp3 provided that it adopts the conformation of native pgp3. It may therefore be a fragment of the complete pgp3 or a derivative thereof modified in a way which does not substantially affect it functional properties, particularly its immunological properties as an antigen.

Preferably the recombinant protein has its native amino sequence and substantially a native conformation.

According to a second aspect of the invention there is provided an immunodiagnostic assay comprising at least one step involving as at least one binding partner, a recombinant protein according to the first aspect of the invention, optionally labelled or coupled to a solid support.

The immunodiagnostic assay may be any form of immunoassay employing as an essential component a diagnostic antigen.

Preferably however, the immunodiagnostic assay is an enzyme-linked solid phase immunoassay (ELISA).

According to a third aspect of the invention there is provided an immunodiagnosis kit for performing an assay according to second aspect of the invention, comprising at least one recombinant protein according to the first aspect of the invention.

According to a fourth aspect of the invention there is provided a method for the production of a recombinant protein according to the first aspect of the invention, comprising culturing a host cell transformed with a vector comprising a recombinant polynucleotide encoding the recombinant protein according to the first aspect of the invention and isolating the recombinant protein.

The host cell may be any suitable host capable of producing the recombinant protein. Preferably however, the host cell is a bacterium, suitably *E.coli*.

Most preferably the protein is produced by overexpression in an *E.coli* expression system. It often found that expression in *E. coli* gives rise to unnatural folding and here non-native conformation. Such expression requires carefully controlled purification techniques, often requiring denaturation and renaturation to recover native protein.

We have discovered, surprisingly, that, in the case of pgp3, the protein is produced in native form by *E.coli* obviating the need for such procedures which add not only to the production cost, but also gives rise to quality control problems associated with the possibility of inadequate removal of reagent employed in purification, such as strong denaturants.

A particularly suitable expression system is the plasmid expression vector pT7-7 optionally in *E.coli* strain BL21. In this system, the expression of ORF3 is under the control of an IPTG-inducible promoter and yields the unmodified pgp3 amino acid sequence.

Preferably, the protein is purified from host cell extracts under non-denaturing conditions.

Preferably the purification comprises a step of ion-exchange column chromatography on, for example, mono-Q prepacked columns (Pharmacia) employing an NaCl elution gradient in piperazine-HCl buffer.

Extraction from the host cell may employ any known non-denaturing technique, such as cell lysis, but preferably employs a periplasmic extract obtained for example using polymixin B.

We have surprisingly discovered that, in *E.coli* the pgp3 protein is produced in the periplasm, greatly facilitating purification.

It is believed from the experimental evidence we now have that pgp3 is a virulence factor of chlamydial infections.

The fourth aspect of the invention also provides recombinant pgp3 protein produced by the method of the invention as broadly defined or as specifically disclosed in the specific description.

According to a fifth aspect of the invention, we provide a vaccine or therapeutic composition comprising a recombinant protein according to the first aspect of the invention and a pharmaceutically acceptable carrier.

According to a sixth aspect of the invention, we provide a vaccine or therapeutic composition comprising a recombinant protein according to the first aspect of the invention and a pharmaceutical carrier.

According to a seventh aspect of the invention, we provide a method of treatment of the human or animal body comprising administering an effective amount of a vaccine or therapeutic composition according to the sixth aspect of the invention to prevent infection by *C.trachomatis* or to treat such an infection.

According to an eighth aspect of the invention, we provide a recombinant protein of claims 1 or 2 for use in the manufacture of a medicament for vaccinating against *C.trachomatis* infection or treating such an infection.

### Definitions

The practice of the present invention will employ, unless otherwise indicated, conventional techniques of molecular biology, microbiology, recombinant DNA, and immunology, which are within the skill of the art. Such techniques are explained fully in the literature. See e.g., Sambrook, et al., MOLECULAR CLONING; A LABORATORY MANUAL, SECOND EDITION (1989); DNA CLONING, VOLUMES I AND II (D.N Glover ed. 1985); OLIGONUCLEOTIDE SYNTHESIS (M.J. Gait ed, 1984); NUCLEIC ACID HYBRIDIZATION (B.D. Hames & S.J. Higgins eds. 1984); TRANSCRIPTION AND TRANSLATION (B.D. Hames & S.J. Higgins eds. 1984); ANIMAL CELL CULTURE (R.I. Freshney ed. 1986); IMMOBILIZED CELLS AND ENZYMES (IRL Press, 1986); B. Perbal, A PRACTICAL GUIDE TO MOLECULAR CLONING (1984); the series, METHODS IN ENZYMOLOGY (Academic Press, Inc.); GENE TRANSFER VECTORS FOR MAMMALIAN CELLS (J.H. Miller and M.P. Calos eds. 1987, Cold Spring Harbor Laboratory), Methods in Enzymology Vol. 154 and Vol. 155 (Wu and Grossman, and Wu, eds., respectively), Mayer and Walker, eds. (1987), IMMUNOCHEMICAL METHODS IN CELL AND MOLECULAR BIOLOGY (Academic Press, London), Scopes, (1987), PROTEIN PURIFICATION: PRINCIPLES AND PRACTICE, Second Edition (Springer-Verlag, N.Y.), and HANDBOOK OF EXPERIMENTAL IMMUNOLOGY, VOLUMES I-IV (D.M. Weir and C.C. Blackwell eds 1986).

Standard abbreviations for nucleotides and amino acids are used in this specification. All publications ,patents, and patent applications cited herein are incorporated by reference.

Examples of the protein that can be used in the present invention include polypeptides with minor amino acid variations from the natural amino acid sequence of the protein; in particular, conservative amino acid replacements are contemplated. Conservative replacements are those that take place within a family of amino acids that are related in their side chains. Genetically encoded amino acids are generally divided into four families: (1) acidic = aspartate, glutamate; (2) basic = lysine, arginine, histidine; (3) non-polar = alanine, valine, leucine, isoleucine, proline, phenylalanine, methionine, tryptophan; and (4) uncharged polar = glycine, asparagine, glutamine, cystine, serine, threonine, tyrosine. Phenylalanine, tryptophan, and tyrosine are sometimes classified jointly as aromatic amino acids. For example, it is reasonably predictable that an isolated replacement of a leucine with an isoleucine or valine, an aspartate with a glutamate, a threonine with a serine, or a similar conservative replacement of an amino acid with a structurally related amino acid will not have a major effect on the biological activity. Polypeptide molecules having substantially the same amino acid sequence as the protein but possessing minor amino acid substitutions that do not substantially affect the functional aspects are within the definition of the protein.

A significant advantage of producing the protein by recombinant DNA techniques rather than by isolating and purifying a protein from natural sources is that equivalent quantities of the protein can be produced by using less starting material than would be required for isolating the protein from a natural source. Producing the protein by recombinant techniques also permits the protein to be isolated in the absence of some molecules normally present in cells. Indeed, protein compositions entirely free of any trace of human protein contaminants can readily be produced because the only human protein produced by the recombinant non-human host is the recombinant protein at issue. Potential viral agents from natural sources and viral components pathogenic to humans are also avoided.

The term "recombinant polynucleotide" as used herein intends a polynucleotide of genomic, cDNA, semisynthetic, or synthetic origin which, by virtue of its origin or manipulation: (1) is not associated with all or a portion of a polynucleotide with which it is associated in nature, (2) is linked to a polynucleotide other than that to which it is linked in nature, or (3) does not occur in nature.

The term "polynucleotide" as used herein refers to a polymeric form of nucleotides of any length, either ribonucleotides or deoxyribonucleotides. This term refers only to the primary structure of the molecule. Thus, this term includes double- and single-stranded DNA and RNA. It also includes known types of modifications, for example, labels which are known in the art, methylation, "caps", substitution of one or more of the naturally occurring nucleotides with an analog, internucleotide modifications such as, for example, those with uncharged linkages (e.g., methyl phosphonates, phosphotriesters, phosphoamidates, carbamates, etc.) and with charged linkages (e.g., phosphorothioates, phosphorodithioates, etc.), those containing pendant moieties, such as, for example proteins (including for e.g., nucleases, toxins, antibodies, signal peptides, poly-L-lysine, etc.), those with intercalators (e.g., acridine, psoralen, etc.), those containing chelators (e.g., metals, radioactive metals, boron, oxidative metals, etc.), those containing alkylators, those with modified linkages (e.g., alpha anomeric nucleic acids, etc.), as well as unmodified forms of the polynucleotide.

A "replicon" is any genetic element, e.g., a plasmid, a chromosome, a virus, a cosmid, etc. that behaves as an autonomous unit of polynucleotide replication within a cell; i.e., capable of replication under its own control. This may include selectable markers.

A "vector" is a replicon in which another polynucleotide segment is attached, so as to bring about the replication and/or expression of the attached segment.

"Control sequence" refers to polynucleotide sequences which are necessary to effect the expression of coding sequences to which they are ligated. The nature of such control sequences differs depending upon the host organism; in prokaryotes, such control sequences generally include promoter, ribosomal binding site, and transcription termination sequence; in eukaryotes, generally, such control sequences include promoters and transcription termination sequence. The term "control sequences" is intended to include, at a minimum, all components whose presence is necessary for expression, and may also include additional components whose presence is advantageous, for example, leader sequences and fusion partner sequences.

"Operably linked" refers to a juxtaposition wherein the components so described are in a relationship permitting them to function in their intended manner. A control sequence "operably linked" to a coding sequence is ligated in such a way that expression of the coding sequence is achieved under conditions compatible with the control sequences.

An "open reading frame" (ORF) is a region of a polynucleotide sequence which encodes a polypeptide; this region may represent a portion of a coding sequence or a total coding sequence.

A "coding sequence" is a polynucleotide sequence which is translated into a polypeptide, usually via mRNA, when placed under the control of appropriate regulatory sequences. The boundaries of the coding sequence are determined by a translation start codon at the 5'-terminus and a translation stop codon at the 3'-terminus. A coding sequence can include, but is not limited to, cDNA, and recombinant polynucleotide sequences.

"PCR" refers to the technique of polymerase chain reaction as described in Saiki, et al., Nature 324:163 (1986); and Scharf et al., Science (1986) 233:1076-1078; and U.S. 4,683,195; and U.S. 4,683,202. As used herein, x is "heterologous" with respect to y if x is not naturally associated with y in the identical manner; i.e., x is not associated with y in nature or x is not associated with y in the same manner as is found in nature.

"Homology" refers to the degree of similarity between x and y. The correspondence between the sequence from one form to another can be determined by techniques known in the art. For example, they can be determined by a direct comparison of the sequence information of the polynucleotide. Alternatively, homology can be determined by hybridization of the polynucleotides under conditions which form stable duplexes between homologous regions (for example, those which would be used prior to S₁ digestion), followed by digestion with single-stranded specific nuclease(s), followed by size determination of the digested fragments.

As used herein, the term "polypeptide" refers to a polymer of amino acids and does not refer to a specific length of the product; thus, peptides, oligopeptides, and proteins are included within the definition of polypeptide. This term also does not refer to or exclude post expression modifications of the polypeptide, for example, glycosylations, acetylations, phosphorylations and the like. Included within the definition are, for example, polypeptides containing one or more analogs of an amino acid (including, for example, unnatural amino acids, etc.), polypeptides with substituted linkages, as well as other modifications known in the art, both naturally occurring and non-naturally occurring.

A polypeptide or amino acid sequence "derived from" a designated nucleic acid sequence refers to a polypeptide having an amino acid sequence identical to that of a polypeptide encoded in the sequence, or a portion thereof wherein the portion consists of at least 3-5 amino acids, and more preferably at least 8-10 amino acids, and even more preferably at least 11-15 amino acids, or which is immunologically identifiable with a polypeptide encoded in the sequence. This terminology also includes a polypeptide expressed from a designated nucleic acid sequence.

The protein may be used for producing antibodies, either monoclonal or polyclonal, specific to the protein. The methods for producing these antibodies are known in the art.

"Recombinant host cells", "host cells," "cells," "cell cultures," and other such terms denote, for example, microorganisms, insect cells, and mammalian cells, that can be, or have been, used as recipients for recombinant vector or other transfer DNA, and include the progeny of the original cell which has been transformed. It is understood that the progeny of a single parental cell may not necessarily be completely identical in morphology or in genomic or total DNA complement as the original parent, due to natural, accidental, or deliberate mutation. Examples for mammalian host cells include Chinese hamster ovary (CHO) and monkey kidney (COS) cells.

Specifically, as used herein, "cell line," refers to a population of cells capable of continuous or prolonged growth and division in vitro. Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants. The term "cell lines" also includes immortalized cells. Preferably, cell lines include nonhybrid cell lines or hybridomas to only two cell types.

As used herein, the term "microorganism" includes prokaryotic and eukaryotic microbial species such as bacteria and fungi, the latter including yeast and filamentous fungi.

"Transformation", as used herein, refers to the insertion of an exogenous polynucleotide into a host cell, irrespective of the method used for the insertion, for example, direct uptake, transduction, f-mating or electroporation. The exogenous polynucleotide may be maintained as a non-integrated vector, for example, a plasmid, or alternatively, may be integrated into the host genome.

By "genomic" is meant a collection or library of DNA molecules which are derived from restriction fragments that have been cloned in vectors. This may include all or part of the genetic material of an organism.

By "cDNA" is meant a complimentary mRNA sequence that hybridizes to a complimentary strand of mRNA.

By "purified" and "isolated" is meant, when referring to a polypeptide or nucleotide sequence, that the indicated molecule is present in the substantial absence of other biological macromolecules of the same type. The term "purified" as used herein preferably means at least 75% by weight, more preferably at least 85% by weight, more preferably still at least 95% by weight, and most preferably at least 98% by weight, of biological macromolecules of the same type present (but water, buffers, and other small molecules, especially molecules having a molecular weight of less than 1000, can be present).

### Expression Systems

Once the appropriate coding sequence is isolated, it can be expressed in a variety of different expression systems; for example those used with mammalian cells, baculoviruses, bacteria, and yeast.

### i. Mammalian Systems

Mammalian expression systems are known in the art. A mammalian promoter is any DNA sequence capable of binding mammalian RNA polymerase and initiating the downstream (3') transcription of a coding sequence (e.g. structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation [Sambrook et al. (1989) "Expression of Cloned Genes in Mammalian Cells." In Molecular Cloning: A Laboratory Manual, 2nd ed.].

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallotheionein gene, also provide useful promoter sequences. Expression may be either constitutive or regulated (inducible), depending on the promoter can be induced with glucocorticoid in hormone-responsive cells.

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter [Maniatis et al. (1987) Science 236:1237; Alberts et al. (1989) Molecular Biology of the Cell, 2nd ed.]. Enhancer elements derived from viruses may be particularly useful, because they usually have a broader host range. Examples include the SV40 early gene enhancer [Dijkema et al (1985) EMBO J. 4:761] and the enhancer/promoters derived from the long terminal repeat (LTR) of the Rous Sarcoma Virus [Gorman et al. (1982b) Proc. Natl. Acad. Sci. 79:6777] and from human cytomegalovirus [Boshart et al. (1985) Cell 41:521]. Additionally, some enhancers are regulatable and become active only in the presence of an inducer, such as a hormone or metal ion [Sassone-Corsi and Borelli (1986) Trends Genet. 2:215; Maniatis et al. (1987) Science 236:1237].

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in vitro incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either in vivo or in vitro. The leader sequence fragment usually encodes a signal peptide comprised of hydrophobic amino acids which direct the secretion of the protein from the cell. The adenovirus triparite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation [Birnstiel et al. (1985) Cell 41:349; Proudfoot and Whitelaw (1988) "Termination and 3' end processing of eukaryotic RNA. In Transcription and splicing (ed. B.D. Hames and D.M. Glover); Proudfoot (1989) Trends Biochem. Sci. 14:105]. These sequences direct the transcription of an mRNA which can be translated into the polypeptide encoded by the DNA. Examples of transcription terminater/polyadenylation signals include those derived from SV40 [Sambrook et al (1989) "Expression of cloned genes in cultured mammalian cells." In Molecular Cloning: A Laboratory Manual].

Some genes may be expressed more efficiently when introns (also called intervening sequences) are present. Several cDNAs, however, have been efficiently expressed from vectors that lack splicing signals (also called splice donor and acceptor sites) [see e.g., Gothing and Sambrook (1981) Nature 293:620]. Introns are intervening noncoding sequences within a coding sequence that contain splice donor and acceptor sites. They are removed by a process called "splicing," following polyadenylation of the primary transcript [Nevins (1983) Annu. Rev. Biochem. 52:441; Green (1986) Annu. Rev. Genet. 20:671; Padgett et al. (1986) Annu. Rev. Biochem. 55:1119; Krainer and Maniatis (1988) "RNA splicing." In Transcription and splicing (ed. B.D. Hames and D.M. Glover)].

Usually, the above described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g., plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 [Gluzman (1981) Cell 23:175] or polyomavirus, replicate to extremely high copy number in the presence of the appropriate viral T antigen. Additional examples of mammalian replicons include those derived from bovine papillomavirus and Epstein-Barr virus. Additionally, the replicon may have two replicaton systems, thus allowing it to be maintained, for example, in mammalian cells for expression and in a prokaryotic host for cloning and amplification. Examples of such mammalian-bacteria shuttle vectors include pMT2 [Kaufman et al. (1989) Mol. Cell. Biol. 9:946 and pHEBO [Shimizu et al. (1986) Mol. Cell. Biol. 6:1074].

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

### ii. Baculovirus Systems

The polynucleotide encoding the protein can also be inserted into a suitable insect expression vector, and is operably linked to the control elements within that vector. Vector construction employs techniques which are known in the art.

Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, inter alia, Invitrogen, San Diego CA ("MaxBac" kit). These techniques are generally known to those skilled in the art and fully described in Summers and Smith, Texas Agricultural Experiment Station Bulletin No. 1555 (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g., plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, Virology (1989) 17:31.

The plasmid usually also contains the polyhedrin polyadenylation signal (Miller et al. (1988) Ann. Rev. Microbiol., 42:177) and a prokaryotic ampicillin-resistance (amp) gene and origin of replication for selection and propagation in E. coli.

Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: The Molecular Biology of Baculoviruses (ed. Walter Doerfler); EPO Publ. Nos. 127 839 and 155 476; and the gene encoding the p10 protein, Vlak et al., (1988), J. Gen. Virol. 69:765.

DNA encoding suitable signal sequences can be derived from genes for secreted insect or baculovirus proteins, such as the baculovirus polyhedrin gene (Carbonell et al. (1988) Gene, 73:409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human α-interferon, Maeda et al., (1985), Nature 315:592; human gastrin-releasing peptide, Lebacq-Verheyden et al., (1988), Molec. Cell. Biol. 8:3129; human IL-2, Smith et al., (1985) Proc. Nat'l Acad. Sci. USA, 82:8404; mouse IL-3, (Miyajima et al., (1987) Gene 58:273; and human glucocerebrosidase, Martin et al. (1988) DNA, 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by in vitro incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by co-transfection. The promoter and transcription termination sequence of the construct will usually comprise a 2-5kb section of the baculovirus genome. Methods for introducing heterologous DNA into the desired site in the baculovirus virus are known in the art. (See Summers and Smith supra; Ju et al. (1987); Smith et al., Mol. Cell. Biol. (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), Bioessays 4:91.The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 µm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. "Current Protocols in Microbiology" Vol. 2 (Ausubel et al. eds) at 16.8 (Supp. 10, 1990); Summers and Smith, supra; Miller et al. (1989).

Recombinant baculovirus expression vectors have been developed for infection into several insect cells. For example, recombinant baculoviruses have been developed for, inter alia: Aedes aegypti , Autographa californica, Bombyx mori, Drosophila melanogaster, Spodoptera frugiperda, and Trichoplusia ni (PCT Pub. No. WO 89/046699; Carbonell et al., (1985) J. Virol. 56:153; Wright (1986) Nature 321:718; Smith et al., (1983) Mol. Cell. Biol. 3:2156; and see generally, Fraser, et al. (1989) In Vitro Cell. Dev. Biol. 25:225).

Cells and cell culture media are commercially available for both direct and fusion expression of heterologous polypeptides in a baculovirus/expression system; cell culture technology is generally known to those skilled in the art. See, e.g., Summers and Smith supra.

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g., HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g., proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

### iii. Bacterial Systems

Bacterial expression techniques are known in the art. A bacterial promoter is any DNA sequence capable of binding bacterial RNA polymerase and initiating the downstream (3") transcription of a coding sequence (e.g. structural gene) into mRNA. A promoter will have a transcription initiation region which isusually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli (E. coli) [Raibaud et al. (1984) Annu. Rev. Genet. 18:173]. Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (lac) [Chang et al. (1977) Nature 198:1056], and maltose. Additional examples include promoter sequences derived from biosynthetic enzymes such as tryptophan (trp) [Goeddel et al. (1980) Nuc. Acids Res. 8:4057; Yelverton et al. (1981) Nucl. Acids Res. 9:731; U.S. Patent No. 4,738,921; EPO Publ. Nos. 036 776 and 121 775]. The g-laotamase (bla) promoter system [Weissmann (1981) "The cloning of interferon and other mistakes." In Interferon 3 (ed. I. Gresser)], bacteriophage lambda PL [Shimatake et al. (1981) Nature 292:128] and T5 [U.S. Patent No. 4,689,406] promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter [U.S. Patent No. 4,551,433]. For example, the tac promoter is a hybrid trp-lac promoter comprised of both trp promoter and lac operon sequences that is regulated by the lac repressor [Amann et al. (1983) Gene 25:167; de Boer et al. (1983) Proc. Natl. Acad. Sci. 80:21]. Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophase T7 RNA polymerase/promoter system is an example of a coupled promoter system [Studier et al. (1986) J. Mol. Biol. 189:113; Tabor et al. (1985) Proc Natl. Acad. Sci. 82:1074]. In addition, a hybrid promoter can also be comprised of a bacteriophage promoter and an E. coli operator region (EPO Publ. No. 267 851).

In addition to a functioning promoter sequence, an efficient ribosome binding site is also useful for the expression of foreign genes in prokaryotes. In E. coli, the ribosome binding site is called the Shine-Dalgarno (SD) sequence and includes an initiation codon (ATG) and a sequence 3-9 nucleotides in length located 3-11 nucleotides upstream of the initiation codon [Shine et al. (1975) Nature 254:34]. The SD sequence is thought to promote binding of mRNA to the ribosome by the pairing of bases between the SD sequence and the 3' and of E. coli 16S rRNA [Steitz et al. (1979) "Genetic signals and nucleotide sequences in messenger RNA." In Biological Regulation and Development: Gene Expression (ed. R.F. Goldberger)]. To express eukaryotic genes and prokaryotic genes with weak ribosome-binding site [Sambrook et al. (1989) "Expression of cloned genes in Escherichia coli." In Molecular Cloning: A Laboratory Manual].

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by in vitro incubation with cyanogen bromide or by either in vivo on in vitro incubation with a bacterial methionine N-terminal peptidase (EPO Publ. No. 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene [Nagai et al. (1984) Nature 309:810]. Fusion proteins can also be made with sequences from the lacZ [Jia et al. (1987) Gene 60:197], trpE [Allen et al. (1987) J. Biotechnol. 5:93; Makoff et al. (1989) J. Gen. Microbiol. 135:11], and Chey [EPO Publ. No. 324 647] genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (e.g. ubiquitin specific processing-protease) to cleave the ubiquitin from the foreign protein. Through this method, native foreign protein can be isolated [Miller et al. (1989) Bio/Technology 7:698].

Alternatively, foreign proteins can also be secreted from the cell by creating chimeric DNA molecules that encode a fusion protein comprised of a signal peptide sequence fragment that provides for secretion of the foreign protein in bacteria [U.S. Patent No. 4,336,336]. The signal sequence fragment usually encodes a signal peptide comprised of hydrophobic amino acids which direct the secretion of the protein from the cell. The protein is either secreted into the growth media (gram-positive bacteria) or into the periplasmic spece, located between the inner and outer membrane of the cell (gram-negative bacteria). Preferably there are processing sites, which can be cleaved either in vivo or in vitro encoded between the signal peptide fragment and the foreign gene.

DNA encoding suitable signal sequences can be derived from genes for secreted bacterial proteins, such as the E. coli outer membrane protein gene (ompA) [Masui et al. (1983), in: Experimental Manipulation of Gene Expression; Ghrayeb et al. (1984) EMBO J. 3:2437] and the E. coli alkaline phosphatase signal sequence (phoA) [Oka et al. (1985) Proc. Natl. Acad. Sci. 82:7212]. As an additional example, the signal sequence of the alpha-amylase gene from various Bacillus strains can be used to secrete heterologous proteins from B. subtilis [Palva et al. (1982) Proc. Natl. Acad. Sci. USA 79:5582; EPO Publ. No. 244 042].

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the trp gene in E. coli as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (e.g., plasmids) capable of stable maintenance in a host, such as bacteria. The replicon will have a replication system, thus allowing it to be maintained in a prokaryotic host either for expression or for cloning and amplification. In addition, a replicoin may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bactedrial chromosome. For example, integrating vectors constructed with DNA from various Bacillus strains integrate into the Bacillus chromosome (EPO Publ. No. 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline [Davies et al. (1978) Annu. Rev.Microbiol. 32:469]. Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extrachromosomal replicons or integrating vectors, have been developed for transformation into many bacteria. For example, expression vectors have been developed for, inter alia, the following bacteria: Bacillus subtilis [Palva et al. (1982) Proc. Natl. Acad. Sci. USA 79:5582; EPO Publ. Nos. 036 259 and 063 953; PCT Publ. No. WO 84/04541], Escherichia coli [Shimatake et al. (1981) Nature 292:128; Amann et al. (1985) Gene 40:183; Studier et al. (1986) J. Mol. Biol. 189:113; EPO Publ. Nos. 036 776, 136 829 and 136 907], Streptococcus cremoris [Powell et al. (1988) Appl. Environ. Microbiol. 54:655]; Streptococcus lividans [Powell et al. (1988) Appl. Environ. Microbiol. 54:655], Streptomyces lividans [U.S. Patent No. 4,745,056].

Methods of introducing exogenous DNA into bacterial hosts are well-known in the art, and usually include either the transformation of bacteria treated with CaCl₂ or other agents, such as divalent cations and DMSO. DNA can also be introduced into bacterial cells by electroporation. Transformation procedures usually vary with the bacterial species to be transformed. See e.g., [Masson et al. (1989) FEMS Microbiol. Lett. 60:273; Palva et al. (1982) Proc. Natl. Acad. Sci. USA 79:5582; EPO Publ. Nos. 036 259 and 063 953; PCT Publ. No. WO 84/04541, Bacillus], [Miller et al. (1988) Proc. Natl. Acad. Sci. 85:856; Wang et al. (1990) J. Bacteriol. 172:949, Campylobacter], [Cohen et al. (1973) Proc. Natl. Acad. Sci. 69:2110; Dower et al. (1988) Nucleic Acids Res. 16:6127; Kushner (1978) "An improved method for transformation of Escherichia coli with ColE1-derived plasmids. In Genetic Engineering: Proceedings of the International Symposium on Genetic Engineering (eds. H.W. Boyer and S. Nicosia); Mandel et al. (1970) J. Mol. Biol. 53:159; Taketo (1988) Biochim. Biophys. Acta 949:318; Escherichia], [Chassy et al. (1987) FEMS Microbiol. Lett. 44:173 Lactobacillus]; [Fiedler et al. (1988) Anal. Biochem 170:38, Pseudomonas]; [Augustin et al. (1990) FEMS Microbiol. Lett. 66:203, Staphylococcus], [Barany et al. (1980) J. Bacteriol. 144:698; Harlander (1987) "Transformation oStreptococcus lactis by electroporation, in: Streptococcal Genetics (ed. J. Ferretti and R. Curtiss III); Perry et al. (1981) Infec. Immun. 32:1295; Powell et al. (1988) Appl. Environ. Microbiol. 54:655; Somkuti et al. (1987) Proc. 4th Evr. Cong. Biotechnology 1:412, Streptococcus].

### iv. Yeast Expression

Yeast expression systems are also known to one of ordinary skill in the art. A yeast promoter is any DNA sequence capable of binding yeast RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EPO Publ. No. 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO Publ. No. 329 203). The yeast PHO5 gene, encoding acid phosphatase, also provides useful promoter sequences [Myanohara et al. (1983) Proc. Natl. Acad. Sci. USA 80:1].

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (U.S. Patent Nos. 4,876,197 and 4,880,734). Other examples of hybrid promoters include promoters which consist of the regulatory sequences of either the ADH2, GAL4, GAL10, OR PHO5 genes, combined with the transcriptional activation region of a glycolytic enzyme gene such as GAP or PyK (EPO Publ. No. 164 556). Furthermore, a yeast promoter can include naturally occurring promoters of non-yeast origin that have the ability to bind yeast RNA polymerase and initiate transcription. Examples of such promoters include, inter alia, [Cohen et al. (1980) Proc. Natl. Acad. Sci. USA 77:1078; Henikoff et al. (1981) Nature 283:835; Hollenberg et al. (1981) Curr. Topics Microbiol. Immunol. 96:119; Hollenberg et al. (1979) "The Expression of Bacterial Antibiotic Resistance Genes i the Yeast Saccharomyces cerevisiae," in: Plasmids of Medical, Environmental and Commercial Importance (eds. K>N> Timmis and A. Puhler); Mercerau-Puigalon et al. (1980) Gene 11:163; Panthier et al. (1980) Curr. Genet. 2:109;].

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by in vitro incubation with cyanogen bromide.

Fusion proteins provide an alternative for yeast expression systems, as well as in mammalian, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g., EPO Publ. No. 196 056. Another example is a ubiquitin fusion protein. Such a fusion protein is made with the ubiquitin region that preferably retains a site for a processing enzyme (e.g. ubiquitin-specific processing protease) to cleave the ubiquitin from the foreign protein. Through this method, therefore, native foreign protein can be isolated (see, e.g., PCT Publ. No. WO 88/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either in vivo or in vitro. The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EPO Publ. No. 012 873; JPO Publ. No. 62,096,086) and the A-factor gene (U.S. Patent No. 4,588,684). Alternatively, leaders of non-yeast origin, such as an interferon leader, exist that also provide for secretion in yeast (EPO Publ. No. 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (U.S. Patent Nos. 4,546,083 and 4,870,008; EPO Publ. No. 324 274). Additional leaders employing an alpha-factor leader fragment that provides for secretion include hybrid alpha-factor leaders made with a presequence of a first yeast, but a pro-region from a second yeast alphafactor. (See e.g., PCT Publ. No. WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 [Botstein et al. (1979) Gene 8:17-24], pCl/1 [Brake et al. (1984) Proc. Natl. Acad. Sci USA 81:4642-4646], and YRp17 [Stinchcomb et al. (1982) J. Mol. Biol. 158:157]. In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See e.g., Brake et al., supra.

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome [Orr-Weaver et al. (1983) Methods in Enzymol. 101:228-245]. An integrating vector may be directed to a specific locus in yeast by selecting the appropriate homologous sequence for inclusion in the vector. See Orr-Weaver et al., supra. One or more expression construct may integrate, possibly affecting levels of recombinant protein produced [Rine et al. (1983) Proc. Natl. Acad. Sci. USA 80:6750]. The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as ADE2, HIS4, LEU2, TRP1, and ALG7, and the G418 resistance gene, which confer resistance in yeast cells to tunicamycin and G418, respectively. In addition, a suitable selectable marker may also provide yeast with the ability to grow in the presence of toxic compounds, such as metal. For example, the presence of CUP1 allows yeast to grow in the presence of copper ions [Butt et al. (1987) Microbiol. Rev. 51:351].

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors have been developed for, inter alia, the following yeasts:Candida albicans [Kurtz, et al. (1986) Mol. Cell. Biol. 6:142], Candida maltosa [Kunze, et al. (1985) J. Basic Microbiol. 25:141]. Hansenula polymorpha [Gleeson, et al. (1986) J. Gen. Microbiol. 132:3459; Roggenkamp et al. (1986) Mol. Gen. Genet. 202:302], Kluyveromyces fragilis [Das, et al. (1984) J. Bacteriol. 158:1165], Kluyveromyces lactis [De Louvencourt et al. (1983) J. Bacteriol. 154:737; Van den Berg et al. (1990) Bio/Technology 8:135], Pichia guillerimondii [Kunze et al. (1985) J. Basic Microbiol. 25:141], Pichia pastoris [Cregg, et al. (1985) Mol. Cell. Biol. 5:3376; U.S. Patent Nos. 4,837,148 and 4,929,555], Saccharomyces cerevisiae [Hinnen et al. (1978) Proc. Natl. Acad. Sci. USA 75:1929; Ito et al. (1983) J. Bacteriol. 153:163], Schizosaccharomyces pombe [Beach and Nurse (1981) Nature 300:706], and Yarrowia lipolytica [Davidow, et al. (1985) Curr. Genet. 10:380471 Gaillardin, et al. (1985) Curr. Genet. 10:49].

Methods of introducing exogenous DNA into yeast hosts are well-known in the art, and usually include either the transformation of spheroplasts or of intact yeast cells treated with alkali cations. Transformation procedures usually vary with the yeast species to be transformed. See e.g., [Kurtz et al. (1986) Mol. Cell. Biol. 6:142; Kunze et al. (1985) J. Basic Microbiol. 25:141; Candida]; [Gleeson et al. (1986) J. Gen. Microbiol. 132:3459; Roggenkamp et al. (1986) Mol. Gen. Genet. 202:302; Hansenula]; [Das et al. (1984) J. Bacteriol. 158:1165; De Louvencourt et al. (1983) J. Bacteriol. 154:1165; Van den Berg et al. (1990) Bio/Technology 8:135; Kluyveromyces]; [Cregg et al. (1985) Mol. Cell. Biol. 5:3376; Kunze et al. (1985) J. Basic Microbiol. 25:141; U.S. Patent Nos. 4,837,148 and 4,929,555; Pichia]; [Hinnen et al. (1978) Proc. Natl. Acad. Sci. USA 75;1929; Ito et al. (1983) J. Bacteriol. 153:163 Saccharomyces]; [Beach and Nurse (1981) Nature 300:706; Schizosaccharomyces]; [Davidow et al. (1985) Curr. Genet. 10:39; Gaillardin et al. (1985) Curr. Genet. 10:49; Yarrowia].

### Nucleic Acid Assays

Polynucleotide probes of approximately 8 nucleotides or more can be prepared which hybridize with the positive strand(s) of the RNA or its complement, as well as to cDNAs. These polynucleotides serve as probes for the detection, isolation and/or labeling of polynucleotides which contain nucleotide sequences, and/or as primers for the transcription and/or replication of the targeted sequences. Each probe contains a targeting polynucleotide sequence, which is comprised of nucleotides which are complementary to a target nucleotide sequence; the sequence is of sufficient length and complementarily with the sequence to form a duplex which has sufficient stability for the purpose intended. For example, if the purpose is the isolation, via immobilization, of an analyte containing a target sequence, the probes will contain a polynucleotide region which is of sufficient length and complementarily to the targeted sequence to afford sufficient duplex stability to immobilize the analyte on a solid surface under the isolation conditions. For example, also, if the polynucleotide probes are to serve as primers for the transcription and/or replication of target sequences, the probes will contain a polynucleotide region of sufficient length and complementarily to the targeted sequence to allow for replication. For example, also, if the polynucleotide probes are to be used as label probes, or are to bind to multimers, the targeting polynucleotide region would be of sufficient length and complementarily to form stable hybrid duplex structures with the label probes and/or multimers to allow detection of the duplex. The probes may contain a minimum of about 4 contiguous nucleotides which are complementary to the targeted sequence; usually the oligomers will contain a minimum of about 8 continuous nucleotides which are complementary to the targeted sequence, and preferably will contain a minimum of about 14 contiguous nucleotides which are complementary to the targeted sequence.

The probes, however, need not consist only of the sequence which is complementary to the targeted sequence. They may contain additional nucleotide sequences or other moieties. For example, if the probes are to be used as primers for the amplification of sequences via PCR, they may contain sequences which, when in duplex, form restriction enzyme sites which facilitate the cloning of the amplified sequences. For example, also, if the probes are to be used as "capture probes" in hybridization assays, they will be coupled to a "binding partner" as defined above. Preparation of the probes is by means known in the art, including, for example, by methods which include excision, transcription or chemical synthesis.

### Immunodiagnostic Assays

Antigens can be used in immunoassays to detect antibody levels (or conversely antibodies can be used to detect antigen levels) and correlation can be made with disease. Immunoassays based on well defined, recombinant antigens can be developed to replace the invasive diagnostics methods that are used today. Antibodies to proteins within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, etc.) required for the conduct of the assay, as well as suitable set of assay instructions.

### Vaccines

Vaccines may either be prophylactic (to prevent infection) or therapeutic (to treat disease after infection).

Such vaccines comprise antigen or antigens, usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the antigen may be conjugated to a bacterial toxoid, such as a toxoid from diphtheria, tetanus, cholera, H. pylori, etc. pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freunds Adjuvant (CFA) and Incomplete Freunds Adjuvant (IFA); (5) cytokines, such as interleukins (IL-1, IL-2, etc.), macrophage colony stimulating factor (M-CSF), tumor necrosis factor (TNF), etc; and (6) other substances that act as immunostimulating agents to enhance the effectiveness of the composition.

Alum and MF59 are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-huydroxyphosphoryloxy)-ethylamine (MTP-PE), etc.

The immunogenic compositions (e.g., the antigen, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles.

Typically, the immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

Immunogenic compositions used as vaccines comprise an immunologically effective amount of the antigenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (e.g., nonhuman primate, primate, etc.), the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

The immunogenic compositions are conventionally administered parenterally, e.g., by injection, either subcutaneously or intramuscularly. Additional formulations suitable for other modes of administration include oral and pulmonary formulations, suppositories, and transdermal applications. Dosage treatment may be a single dose schedule or a multiple dose schedule. The vaccine may be administered in conjunction with other immunoregulatory agents.

### Brief Description of the Drawings

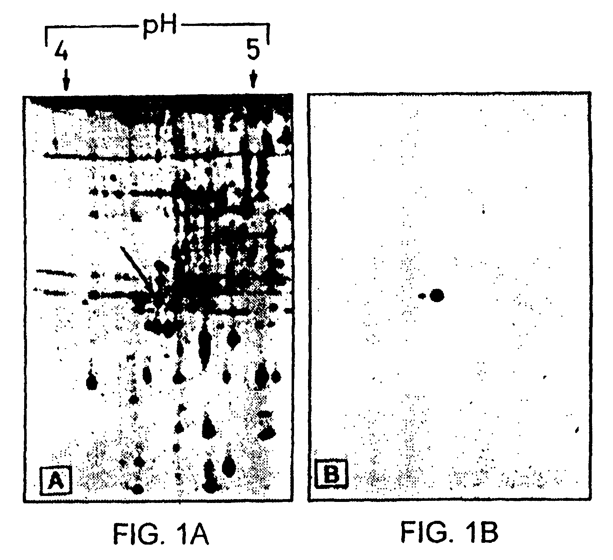

Figure 1A shows part of a silver-stained gel showing chlamydial EB proteins with Mᵣ between 25,000 and 40,000 and pI values between 4 and 5 (non-linear pH gradient). EBs. The arrow shows the spot which was eluted for N-terminal amino acid sequencing.
Figure 1B shows part of an immunoblot of the map region shown on the left, developed with a pgp3-specific rabbit serum.

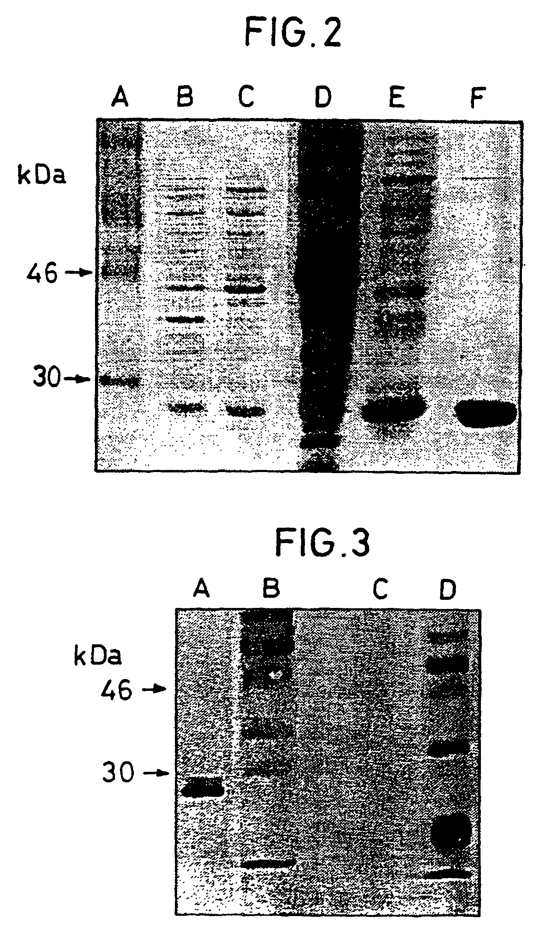

Figure 2 shows Coomassie Blue stained gels after SDS-PAGE following expression in *E.coli* BL21(DE3), and purification of r-pgp3. **Lane A:** size markers. **Lanes B, C:** pellet (B) and supernatant (C) fractions of pgp3-expressing *E.coli* cells after Polymyxin B treatment and centrifugation. **Lanes D, E:** pellet (D) and supernatant (E) samples of periplasmic fractions (as in lane C), after dialysis against piperazine-HCl buffer pH 5.4, and centrifugation. **Lane F:**pooled peak-fractions after ion-exchange chromatography.

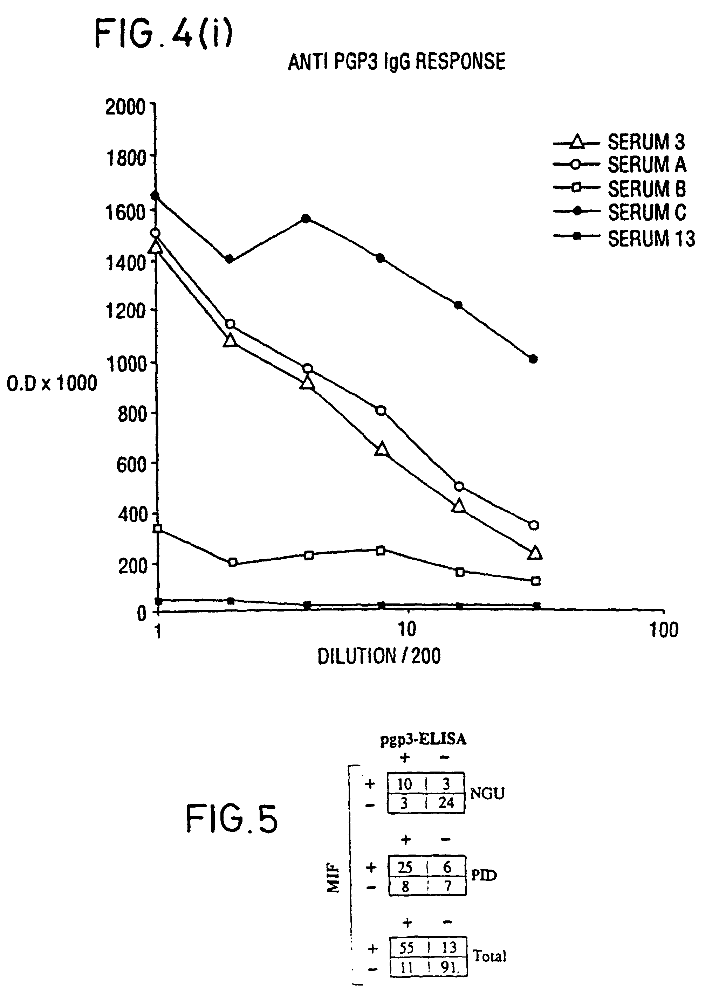

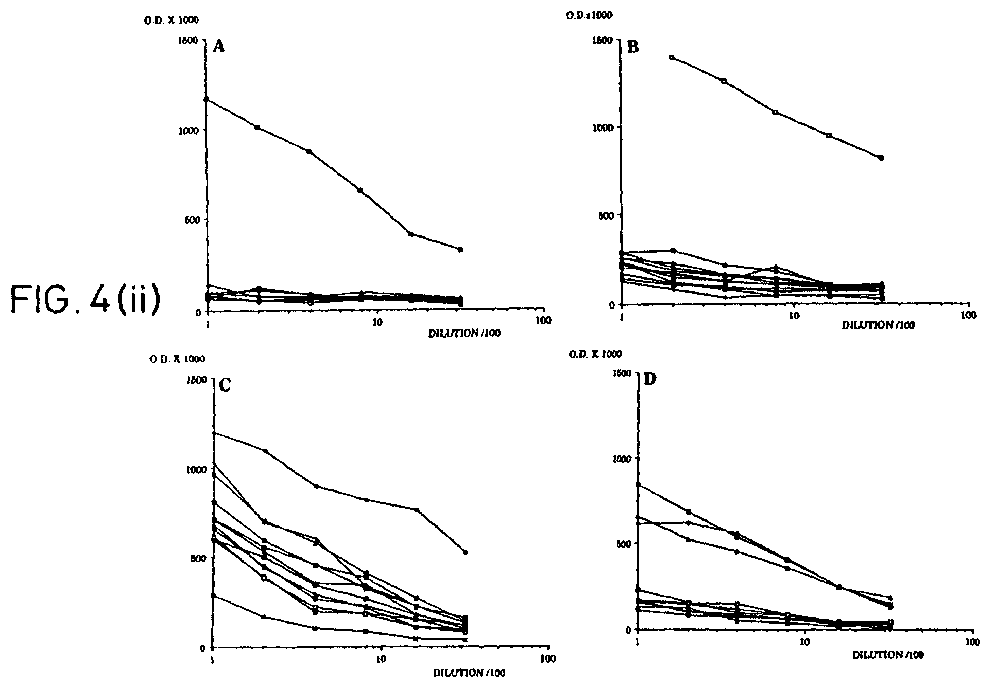

Figure 3 shows immunoblot analysis of human sera with purified r-pgp3. Typical positive and negative results (**A** and **C**) are shown. Immunoblots with crude *E.coli* extracts are also ,shown (**B** and **D**): these gave variable patterns of reactivity to *E.coli* antigens, served for patient identification, and as a positive control for negative samples. **Blots A and B:** serum of a patient affected by salpingitis and MIF-positive for *C.trachomatis*. The ELISA response of this serum is shown in Fig.4C (curve with intermediate response). **Blots C and D:** serum from a healthy blood donor, MIF-negative for *C.trachomatis*. This serum was used as a negative ELISA control in the present study.
Figure 4(i) is a graph showing the reaction between plastic bound recombinant pgp3 protein of the present invention and various human sera. Sera C, A and 3 were from women with salpingitis. Serum B was from a male with isolation-positive chlamydial urethitis and serum 13 was from a healthy blood donor.
Figure 4(ii) shows typical positive and negative pgp3-ELISA results. Semilogarithmic plots of OD readings (each point is the average value of duplicate samples) against 2-fold dilutions of the sera, starting from 1/100. **A:** 10 healthy blood donor sera which gave negative MIF results with purified chlamydial EBs of all three species, and did not react with r-pgp3 on immunoblots. The upper curve (dotted squares) was given by a positive control serum (immunoblot-positive, MIF-positive) assayed on the same microtiter plate. **B:** 10 sera from patients with *C. pneumoniae* infection (MIF >512). The upper curve (open squares) is the positive control. These results are comparable to those obtained with healthy subjects in panel A. **C**: positive pgp3-ELISA results obtained with 10 of 46 salpingitis sera examined; positive and negative control sera (top and bottom curves, respectively) are included. **D:** 5 negative (curves with OD < 0.25), and 2 positive pgp3-ELISA results (solid diamonds and triangles) obtained with 7 of 40 male urethritis sera examined. A positive (dotted squares) and negative (open triangles) control are also shown.
Figure 5 shows the prevalence of anti-pgp3 IgGs in human sera and comparison with anti-EB surface IgGs (MIF) prevalence. **Top panel:** results obtained with a group of 40 male urethritis (NGU) sera. **Middle panel:** results obtained with 46 salpingitis sera. For convenience, the more general term PID (for pelvic inflammatory disease) has been used in the table. **Bottom panel:** summary of all results, including those from the 10 *C.pneumoniae* positive sera, 50 healthy blood donor sera and results obtained with a group of 24 sera from women with various symptoms of genital tract infection or secondary sterility.

### Detailed Description of Embodiments

The examples presented herein are provided as a further guide to the practitioner of ordinary skill in the art and are not to be construed as limiting the invention in any way.

### Production of recombinant pgp3 in E.coli.

ORF3 DNA (pCT segment from bp 4054 to bp 5013, according to ref.3) was obtained by a polymerase chain reaction (PCR) (22) using 10 ng of plasmid pUC8-pCTD (3), as a template, and 20 pmoles each of the following primers: 5'-GGGcatatgGGAAATTCTGGTTTTT-3', and

5'-CCCctgcagAAAGTTACTTTTTCCTTG-3'.

Taq DNA polymerase and the GeneAmp kit (Perkin-Elmer) were used according to manufacturer's recommendations. Primers were designed with *Nde*I and *Pst*I restriction sites (low case characters in the above sequences) at their 5' ends, in order to orient the amplified DNA fragment into the corresponding sites of expression plasmid vector pT7-7 (29, 30, 35). The PCR product was purified using Centricon cartridges, digested with *Nde*I/*Pst*I endonucleases (Boehringer) and ligated into pT7-7. The ligase reaction mix was used to transform *E.coli* strain DH5 (7) which was plated on LB agar (23) containing 100 µg/ml ampicillin. Colonies were screened for the presence of the ORF3 insertion by hybridization with ORF3-specific synthetic oligonucleotides, end labelled with ³²P (23). DNA from positive colonies were then used to transform *E.coli* BL21(DE3) competent cells (29, 30) which were selected first on ampicillin-LB agar plates (23) and then by their capability of expressing the recombinant protein.

Bacteria from positive colonies were grown overnight in 10 ml of LB medium containing 100 µg/ml ampicillin. Overnight cultures were diluted (1:200) with fresh LB medium without ampicillin and grown at 37 °C until O.D.₅₉₀= 0.6. Since expression in the pT7-7/BL21(DE3) system is under the control of an IPTG-inducible promoter, ORF3 expression was achieved by adding 0.4mM IPTG to the medium and further incubating for 2.5 hours with vigorous shaking. Bacterial cells, collected by centrifugation were resuspended in 1/20 of the initial volume of 25% Sucrose, 50mM Tris-Hcl, pH 8, containing 1 mg/ml of polymyxin B sulfate (Sigma Chemicals)(18) and incubated at room temperature for 2 hr. After centrifugation (10 min in an Eppendorf centrifuge) most of r-pgp3 was found in the supernatant (periplasmic fraction). The integrity of the bacterial cells was checked by ensuring that the cytoplasmic beta-galactosidase activity (14) remained all in the pellet.

r-pgp3 was detected by polyacrylamide gel electrophoresis, using 12.5% polyacrylamide, 1% SDS gels (SDS-PAGE) according to Laemmli (12). Gels were stained with Coomassie brilliant blue (0.05% w/v) in 10% (v/v) acetic acid, 30% (v/v) methanol. Immunoblot analysis (31) was performed with a pgp3-specific rabbit serum, as described (3). One expression competent *E.coli* clone was eventually selected for further work. The recombinant pT7-7 construct harboured by this clone was checked by sequencing, using the dideoxy terminators technique (25) and the Sequenase kit (US Biochemicals) the entire ORF3 DNA insert, which was identical to the sequence originally described for ORF3.

### Purification of recombinant pqp3

Crude periplasmic fractions were obtained, as described above, from 40 ml or 200 ml bacterial cultures,and dialyzed against 30mM piperazine-HCl, pH 5.4. This caused extensive protein precipitation, but left r-pgp3 in solution. Further purification was obtained by ion-exchange chromatography on mono-Q prepacked columns (Pharmacia) in the same piperazine-HCl buffer. Selective elution was obtained with a NaCl concentration gradient 0 to 1M. FPLC equipment (Pharmacia) was used. In a typical run 4 mg of total protein were loaded and 1ml fractions were collected, and analyzed by SDS-PAGE followed by Coomassie blue staining and immunoblot analysis, as above described. Peak fractions containing purified pgp3 were pooled and dialyzed against sterile PBS (10mM-sodium phosphate pH 7.4, 15mM NaCl). pgp3 purity was estimated as >90% by total protein determination (Biorad Protein Assay) and PAGE together with protein standards (increasing amounts of titrated bovine serum albumin solution) followed by Coomassie Blue staining and photodensimetry measurements using an Ultroscan XL Densitometer (LKB).

### Two dimensional electrophoretic analysis of EB proteins

Large scale preparations of *C.trachomatis* L2/343/Bu elementary bodies (EB) were obtained from Vero cells cultures in rolling bottles, according to described methods (1). EBs were purified by two cycles of density gradient centrifugation (1) and stored at -20 °C for subsequent electrophoretic analysis.

Two-dimensional gel electrophoresis was performed using the immobiline/ polyacrylamide system, essentially as described by Hochstrasser et al. (1988), and Hughes et al. (1992).

Approximately 45 µg (analytical run) or 1mg (preparative run) of total EB protein were used for each run. EBs were pelleted by low speed centrifugation and resuspended in 8M urea, 4% CHAPS ( 3-[(3 cholamidopropyl)dimethylammonium]-1-propanesulfonate), 40mM Tris base, 65mM dithioerythritol (DTE) and trace amounts of Bromophenol Blue. The first dimension was carried out on immobiline strips providing a non-linear pH gradient (IPG strips, Pharmacia) ranging from pH value 3 to 10. Voltage was linearly increased from 300 to 3500 V during the first three hours, then stabilized at 5000 V for 22 hours (total Volt.hour product 110 kVh ). After electrophoresis, IPG strips were equilibrated for 12 min against 6M urea, 30% glycerol, 2% SDS, 0.05M Tris.HCl, pH 6.8, 2% DTE, and subsequently for 5 min in the same urea/SDS/Tris buffer solution but substituting the 2% DTE with 2.5% iodoacetamide. The second dimension was carried out on 9-16% polyacrylamide linear gradient gels (18 cm x 20 cm x 1.5 mm), at 40 mA/gel constant current, for approximately 5 h until the dye front reached the bottom of the gel. Analytical gels were stained with ammoniacal silver nitrate, as described (9, 17).

The pH gradient was monitored with carbamylated creatine kinase (CPK standard, B.D.H.) and corrected, at the non-linear acidic end, according to internal EB protein reference spots, which were identified by immunoblotting with monoclonal antibodies specific for known chlamydial proteins (a gift from G. Christiansen and S. Birkelund)

### N-terminal sequencing of the native 28 kDa chlamydial antigen

For N-terminal primary structure determination, protein spots were electroeluted from the gels onto polyvinylidene difluoride membranes (BioRad PVDF membranes 20 x 20 cm, 0.2 micron pore size)) according to Matsudaira (1987). Blots were stained with 0.1% (w/v) Coomassie Brilliant Blue R250 in 50% aqueous methanol for 5 minutes, and destained in 40% methanol, 10% acetic acid. Membranes were dried at 37 °C (24) and stored at -20 °C for further analysis. The membrane area containing the main pgp3 protein spot, was excised from five identical blots, and the pooled material was submitted to Edman degradation using an automatic Protein/Peptide Sequencer (mod 470A; Applied Biosystem Inc.) connected on-line with a phenylthiohydantoin-amino acid analyzer model 120A and a control/Data Module model 900A (Applied Biosystems Inc.).

### Enzyme linked immunoassay

The purified pgp3 antigen was used to set up an enzyme linked immunosorbent assay (ELISA) test. 200 ng of protein were adsorbed onto plastic wells of Maxisorp microtiter plates (NUNC) in 100 µl of coating buffer (PBS pH 8.0, 0.005% Tween 20, 0.02% NaN3), first for 2 hours at 37 °C and then overnight at 4 °C. After washing with coating buffer, the wells were saturated with 200 µl of 2.7% Polyvinylpyrrolidone for 2 hours and washed again. 100 µl of serum samples, diluted in coating buffer as required, were added to individual wells and incubated for 2 hours at 37 °C. After repeated washings, bound serum-antibodies were detected by incubation with alkaline phosphatase labelled anti-rabbit or anti-human IgG antibody (Cappel), diluted in coating buffer 1:5000, at 37 °C for 2 hours. For colorimetric detection of enzyme activity ELISA Substrate and Buffer (Sclavo Diagnostics srl) were used as recommended by the manufacturer. Optical density readings were performed, usually after 1 hr of color development, with a Multiscan MCC densitometer (Titertek).

### Microimmunofluorescence

Twofold dilutions of sera were titrated by single-antigen micro-immunofluorescence (MIF) (33) using sucrose-gradient purified EBs of *C.trachomatis* L2/434/Bu and serotype D strain Go/86, *C.psittaci* 6BC and A22, and *C.pneumoniae* IOL-207. Fluorescein conjugated rabbit anti-human IgGs (Dako) were used for detection with a UV microscope (Zeiss). Sera for which possible cross reaction between *C.trachomatis* and *C.pneumoniae* antibodies was suspected, were re-assessed using a commercially available kit which discriminates anti-*C.trachomatis* from anti-*C.peumoniae* response (Immunocomb, PBS Orgenics, Israel). In this case, titers were deduced from a single dilution (1:200) readings, according to manufacturer recommendations.

### Clinical samples

Sera from patients who underwent laparoscopical examination for suspected salpingitis were collected at the Centre Hospitalo-Universitaire, University of Picardie, Amiens, France. Other sera were obtained from women attending the Laboratoire Départemental de la Somme, Amiens, because of symptoms of genital tract infection. A third group of sera was selected from the collection of the Biobanque de Picardie, Amiens; these included sera from healthy seronegative subjects, the group of *C.pneumoniae* positive sera, and sera from volunteers who accepted to submit to periodic clinical examinations at the Centre de Prévention et d'examen de santé d'Amiens. All the above sera were aliquoted in 200 microliter resin straws, and stored at -80 °C, at the Biobanque de Picardie. Healthy blood donor, and male urethritis sera from patients attending the STD clinic of St Orsola Hospital, Bologna were collected by the Institute of Microbiology, Bologna University, Bologna, Italy. For the male urethritis patients, exclusion of gonococcal infection, and *C.trachomatis* isolation from urethral swabs (positive in 50% of cases), were performed as previously described (19).

### Identification of native pgp3

Elementary bodies of *C.trachomatis* L2/434/Bu were grown in Vero cell cultures, and purified by two cycles of density gradient centrifugation. Aliquots of this preparation (45 µg or 1 mg of total protein for analytical or preparative runs, respectively) were dissolved in urea/CHAPS/Tris/DTE solution and charge-fractionated by electrophoresis on IPG immobiline strips. After reaching isoelectric equilibrium, the IPG strips were equilibrated with a new buffer, treated with buffered 2.5% iodoacetamide and loaded on 18x20 cm polyacrylamide gels for protein separation according to size. Two gels were prepared under identical conditions (initial loading 0.05 mg of total EB protein): the first was silver stained, and the second one was electroblotted onto a cellulose nitrate membrane and probed with a pgp3-specific rabbit serum. The silver stained gel showed a pattern of >500 distinct spots in the 10-150 kDa, and pI=3.5-9 ranges. Immunoblot analysis of the EB protein map showed that only two spots were recognized by the anti-pgp3 serum: a major one with coordinates corresponding to Mᵣ=28,000 and pI=4.6, and a minor one of similar Mᵣ, but shifted towards the acidic side of the map (Fig.1B). This minor spot was not further investigated, however, the presence, beside a major protein species, of one or more satellite spots ("charge trains") with same molecular weight, decreasing intensity, and increasing negative charge is a typical pattern which is often observed in two-dimensional electrophoretic maps. These patterns are usually given by progressive deamidation of Asn or Gln residues, generating the corresponding negatively charged acidic residues. By matching the immunoblot with the silver-stained gel, the immunoreactive 28-kDa protein was identified on the map (Fig.lA).

In order to purify this protein for further analysis, 5 mg of total EB protein were separated by two-dimensional electrophoresis on five identical gels (1 mg total protein/gel) and electroblotted onto polyvinylidene membranes. These were then lightly stained with Coomassie Blue and, the major 28-kDa immunoreactive protein spot on each blot was located by pattern comparison with the silver stained protein map and carefully excised. Protein from the six spots was pooled for amino acid sequence analysis. The first 10 N-terminal residues of this protein could be clearly identified in the following sequence: Gly-Asn-Ser-Gly-Phe-Leu-Leu-Tyr-Asn. This sequence is identical to the one previously predicted from ORF3 (2, 3), apart from an initial Met residue, deducible from the translation of the first ATG codon of ORF3 but not present in the protein purified from EBs.

### Expression of ORF3 in E.coli and purification of recombinant pgp3

ORF3 DNA was cloned in the plasmid vector pT7-7 and expressed in *E.coli* BL21 cells under the control of a T7 bacteriophage promoter which can be indirectly activated by the addition of IPTG to the medium. Extracts from a selected *E.coli* BL21 clone were shown, by PAGE analysis, to produce large amounts of recombinant pgp3 (r-pgp3). It was also observed that a large proportion of r-pgp3 was present in the periplasmic fraction which was obtained either by controlled treatment of the bacteria with polymyxin B (ref.18; Fig.2: B and C) or, by osmotic shock (ref 6; data not shown). Since periplasmic extracts had a reduced protein complexity, supernatants obtained by centrifugation of polymyxin-treated BL21 cells (Fig.2C) were used as starting material for further r-pgp3 purification. Preliminary tests showed that ion-exchange chromatography on mono-Q columns (Pharmacia) could be an effective purification procedure. Before loading on mono-Q columns, bacterial extracts were dialyzed against piperazine-HCl buffer, pH 5.4. During dialysis several protein species formed a precipitate, which was removed by centrifugation, while r-pgp3 remained in solution (Fig.2: D and E). Chromatography of the dialyzed extracts was performed in the same piperazine-HCl pH 5.4 buffer and elution with a NaCl concentration gradient. Most r-pgp3 was collected in a major peak with a purity >90%, as judged by PAGE, Coomassie Blue staining and photodensitometry (Fig.2F). Some minor bands in the 80-90 kDa region co-purified with r-pgp3. Since Western blot and ELISA data showed that these contaminants did not generate any appreciable background in the assay of human sera, no further purification was attempted.

### Specific detection of anti-pgp3 antibodies by ELISA

Purified r-pgp3 was initially tested for its ability to bind to NUNC microtiter plate wells. Simple incubation in PBS-0.05% Tween was found to be satisfactory. Rabbit sera, raised either against the 39-kDa fusion protein, or the 28-kDa r-pgp3 here described, were initially used for setting up the assay. Variable amounts (500 ng to 50 ng) of purified antigen in each well were tested against the anti-pgp3 rabbit sera and the amount of 200 ng/well was eventually chosen as a standard assay condition. In order to test if normal human serum components could interfere with the detection of anti-pgp3 antibodies, pgp3-ELISA was tested with selected groups of human sera which were previously analyzed for the presence/absence of anti-chlamydia antibodies (IgGs) by MIF, and for anti-pgp3 IgGs by immunoblot analysis with purified r-pgp3 preparations.

### Experiment 1

In a first experiment, a panel of 21 human sera was used to test the ELISA performance with clinical samples. All sera gave negative MIF response to *C.psittaci* and *C.pneumoniae*. When tested for *C.trachomatis* antibodies, using purified L2-serotype EBs, 15 sera were scored as MIF negative, and 6 positive, with titers comprised between 1:32 and 1:256. These sera were also tested for their ability to react with the purified pgp3 preparation on Western blots: all the MIF negative sera gave negative results, whereas the MIF positive sera reacted, to various extents, with the 28-kDa band only.

Two-fold serial dilutions of the sera were tested, in duplicate samples, with the pgp3-ELISA. OD readings were taken after 30 min, 1 hr and after O/N storage in the cold (-20°C). All 15 negative sera gave consistently low OD readings (below 0.1), whereas the 6 positive sera gave distinctly higher OD readings which proportionally decreased with the serum concentration in the sample. The [OD vs dilution] curves of the positive sera so far tested correlated with the MIF titres.

The results of reacting various human sera at dilutions from 200 to 6800 fold with the plastic-bound recombinant pgp3 protein are represented graphically in Figure 4(i).

ODs are average values obtained from duplicate samples. *C.trachomatis* MIF titres of the sera (top to bottom) were 1:256; 1:128; 1:128; 1:132; zero. Sera C, A and 3 were from women with salpingitis; the serum B was from a male with isolation-positive chlamydial urethritis; serum 13 was from a healthy blood donor.

### Experiment 2

In a second experiment, a panel of 10 human sera giving a MIF positive response to *C.trachomatis* EBs (CT-MIF positive) with titers >64, and 50 healthy blood donor, CT-MIF negative sera were assessed. All CT-MIF positive sera reacted on immunoblots with the r-pqp3 band only, whereas all the CT-MIF negative sera were also immunoblot negative for pgp3. These sera were then assessed by ELISA against r-pgp3 bound to NUNC microtiter plates. Two-fold serial dilutions of each serum were tested, in triplicate samples. OD readings were taken after 1 hr of colour development. All MIF and immunoblot negative sera gave consistently low OD readings (below 0.1, see Fig.4(ii)A), whereas the MIF and immunoblot-positive sera gave variable but consistently higher OD readings which proportionally decreased with the serum dilution in the sample. Considering the high prevalence of antibody against *C.pneumoniae* reported in the literature, we also tested by pgp3-ELISA a group of 10 sera from patients with respiratory symptoms who were MIF-negative for *C.trachomatis*, but had high MIF titers (>512) of antibodies against *C.pneumoniae*. These sera gave pgp3-ELISA responses similar to those obtained with the healthy blood donor control sera (Fig. 4(ii)B). We conclude that cross reactions between pgp3 and antibodies developed in response to *C.pneumoniae* infection are not likely to occur.

### pgp3-ELISA screening of patient sera

In order to evaluate the prevalence of anti-pgp3 antibodies in individuals who have developed, or are developing an immune response to *C.trachomatis* infection, we studied three groups of patients with uro-genital tract inflammation symptoms: 46 female patients with laparoscopically confirmed salpingitis; 24 patients with various conditions often associated to *C.trachomatis* infections (lower genital tract inflammation, secondary sterility); 40 cases of male non-gonococcal urethritis (NGU).
All sera were initially assessed, by experienced staff, at the Hospital of origin by MIF using purified EBs of *C trachomatis*, and *C.pneumoniae*. The 40 NGU cases were re-assessed by MIF after the pgp3-ELISA tests. The 70 female sera, on reception at the Biobanque laboratories, were also assessed with a commercially available, confirmatory test, which efficiently discriminates between *C.trachomatis* and *C.pneumoniae* responses (Orfila et al., unpublished results).

Considering the possibility of cross reactions with immunodominant EB surface components (e.g. LPS) in different chlamydial species (11), sera which gave a positive MIF reaction with *C.trachomatis* EBs but also had equal or higher titers against *C.pneumoniae* EBs were scored according to the confirmatory test: i.e., the sera of this group which the Immunocomb test indicated as positive for *C.pneumoniae* antibodies but negative for *C.trachomatis*, were considered in this study as giving false positive CT-MIF results.

All the above sera were analyzed by pgp3-ELISA on duplicate sets of six twofold dilutions in PBS, from 1:100 to 1:3,200. In general the most informative (i.e. maximal difference between sample and negative control) sample dilutions were those between 1:100 and 1:400. Dose/response curves on semi-logarithmic plots were used to evaluate individual samples. Results were compared with those obtained from positive and negative control sera, which were introduced in each experimental session. Essentially, sera were scored as anti-pgp3 positive when the ELISA readings were consistently greater than those of the negative reference serum for several matching dilution values. Sera yielding OD values consistently lower than two- to threefold the negative controls were considered as negative.

Typical results are shown in Fig. 4(ii)C and 4(ii)D. A summary of CT-MIF and pgp3-ELISA scores is given in Figure 5 and Table I.

**TABLE I**

| Patient group and illness | No of cases | No.(%) pgp3-ELISA positive |
|---|---|---|
| CT-MIF pos., STD patients | | |
| PID | 31 | 25(80.6 %) |
| | | |
| various | 24 | 20(83.3 %0 |
| | | |
| NGU | 13 | 10(76.9 %) |
| CT-MIF neg., STD patients | | |
| PID | 15 | 8(53.3 %) |
| NGU | 27 | 3(11.1 %) |
| CT-MIF neg. | | |
| healthy subjects | 50 | 0(0%) |
| | | |
| CT-MIF neg. | | |
| CPn-MIF positive | 10 | 0(%)0 |

### Table I

Prevalence of pgp3-ELISA positive findings in diverse groups of human sera. As in Figure 5, PID refers to the salpingitis cases. CPn: *Chlamydia pneumoniae*.

The availability of a pgp3-specific ELISA allowed an assessment of the prevalence of humoral anti-pgp3 responses in patients with symptoms of infection of the uro-genital tract. A total of 130 human sera were examined by pgp3-ELISA: for all of them presence or absence of an humoral response to chlamydial surface antigens was assessed by MIF. For 81.5% of all sera examined, MIF and pgp3-ELISA agreed in detecting a positive or negative response to their respective antigens (Figure 5, bottom panel).

Of 110 STD patient sera examined, 68 were CT-MIF positive and 42 were CT-MIF negative; of these, 81% in the first group, and 26% in the second group were also pgp3-ELISA positive. These overall results indicate that most patients which are developing an immune response to *C.trachomatis* surface antigens make also antibodies against pgp3 (p < 0.0005).

If the two groups of STD patients with a relatively well defined pathological condition (NGU and salpingitis) are evaluated, the results show some interesting variation.
The 40 male NGU cases (positive CT-MIF prevalence 32.5%) show an overall anti-pgp3 prevalence of 32.5%, which becomes 76.9% in the CT-MIF positive sub-group. CT-MIF and anti-pgp3 ELISA agree in giving a positive or negative response in 85% of cases (75% for the 20 isolation-positive samples, and 95% for the 20 isolation-negative samples). The statistical correlation between presence of anti-pgp3 antibodies and MIF positivity in the NGU group is again significant (p < 0.0005). For these patients cell culture isolation of viable chlamydia from urethral swabs was also performed and it may be interesting to note that of the 13 ELISA-positive NGU cases, 11 (84.6%) were also culture positive (p=0.007), whereas of the 27 ELISA negative cases 9 (33.3%) were culture positive and 18 (66.6%) were culture negative.
The group of 46 salpingitis cases (positive CT-MIF prevalence 67.4%) show an anti-pgp3 antibody prevalence of 71.7%, which becomes 80.6% in the CT-MIF positive sub-group.

For this group of sera the agreement between CT-MIF and anti-pgp3 ELISA in giving a positive or negative response was lower (69.6% of samples) than for the rest of sera, and the presence of anti-pgp3 antibodies did not correlate well with MIF positivity (p=0.115).

Eleven (26%) of the 42 CT-MIF negative STD patients were positive for the pgp3-ELISA test: of these 8 (53.3%) belonged to the salpingitis group and 3 (11.1%) to the NGU group. Such antibody pattern could be given, in some cases, by differences in levels and persistence of anti-EB-surface versus anti-pgp3 antibodies. Alternatively pgp3-ELISA could detect cross reactions with antibodies generated by infection with microorganisms other than chlamydia or by other pathological conditions, like autoimmunity. The probability of obtaining positive pgp3-ELISA results due to specific cross-reactions needs to be evaluated in further studies on larger and well defined populations; however, the results obtained with animal preimmune sera (not shown) and with 60 CT-MIF negative human sera (healthy, or non-STD patient groups) indicate that pgp3-ELISA false positive results due to aspecific binding of normal serum components are not likely to occur, since none of these sera yielded a positive response.

In contrast, thirteen (19%) of the 68 STD patient sera which were CT-MIF positive did not show detectable levels of anti-pgp3 IgGs. This group may comprise patients with a recent first-time infection who have already developed an anti EB-surface response but not yet a significant response to pgp3. In fact, 2 sera from volunteers who submit to regular controls, and which were surely collected after a recent infection, belong to this group. Another possibility is that some patients may not develop any anti-pgp3 response because of some peculiarity of their immune response to the chlamydial infection.

The purpose of the serological survey here presented is to show that sera of patients with a *C.trachomatis*-related disease recognize pgp3; however, further epidemiological studies may better assess the frequence and significance of such antibody patterns. Also, established animal models of chlamydial infection could be usefully employed to assess the relative timing of appearance of anti-pgp3 and anti-EB surface antibodies.

The function and role of pgp3 in the chlamydial cell and life cycle are still unknown; however, since chlamydia induced disease is thought to be largely determined by the host immune responses to the infection, the data here reported show that pgp3 is one of several molecules which are potentially important for the pathogenicity of chlamydial infections of the urogenital tract.

It will be understood that the invention is described above by way of example only and variations are possible within the spirit and scope of the invention.

### References

1. **Caldwell, H.D., J. Kromhout, and J. Schachter. 1981.** Purification and partial characterization of the major outer membrane protein of *Chlamydia trachomatis*. Infect. Immun. **31:**1161-1176.
2. **Comanducci, M., 8. Ricci, and G. Ratti.** 1988. The structure of a plasmid of *Chlamydia trachomatis* believed to be required for growth within mammalian cells. Mol. Microbiol. **2:**531-538.
3. **Comanducci, M., S. Ricci, R. Cevenini, and G. Ratti.** 1990. Diversity of the chlamydial common plasmid in biovars with different pathogenicity. Plasmid 23:149-154.
4. **Comanducci, M., R. Cevenini, A. Moroni, M. M. Giuliani, 8. Ricci, V. Scarlato, and G. Ratti.** 1993. Expression of a plasmid gene of *Chlamydia trachomatis* encoding a novel 28 kDa antigen. J. Gen. Microbiol. **139:**1083-1092.
5. **Fahr, M.J., K.S. Sriprakash, and T.P. Hatch.** 1992. Convergent and overlapping transcripts of the Chlamydia trachomatis 7.5-kb plasmid. Plasmid **28:**247-257
6. **Gormley, E. P., B. A. Cantwell, P. J. Barker, R. S. Gilmour, and D. J. McConnell.** 1988. Secretion and processing of the *Bacillus subtilis* endo-β-1,3-1,4-glucanase in *Escherichia coli*. Mol. Microbiol. **2:**813-819.
7. **Hanahan, D.** 1983. Studies on transformation of *Escherichia coli* with plasmids. J. Mol. Biol. **166:**557-580.
8. **Hatt, C., M. E. Ward, and I. N. Clarke.** 1988. Analysis of the entire nucleotide sequence of the cryptic plasmid of *Chlamydia trachomatis* serovar L1. Evidence for involvement in DNA replication. Nucl. Acids Res. **16:**4053-4067.
9. **Hochstrasser D. F., M. G. Harrington, A.C. Hochstrasser, M.J. Miller, and C. R. Merryl.** 1988. Method for increasing the resolution of the two-dimensional protein electrophoresis. Anal. Biochem. **173:**424-435.
10. **Hughes G. J., S. Frutiger, N. Paquet, C. Pasquali, J.C. Sanchez, R. James, J. D. Tissot, B. Bjellqvist, and D. F. Hochstrasser.** 1992. Plasma protein map: an update by microsequencing. Electrophoresis **13:**707-714.
11. **Kern, D. G., M. A. Neill, and J. Schachter.** 1993. A seroepidemiological study of *Chlamydia pneumoniae* in Rhode Island. Evidence of serologic cross-reactivity. Chest **104:**208-213.
12. **Laemmli, U.K.** 1970. Cleavage of structural proteins during the assembly of the head of bacteriophage T4. Nature **227:**680-685.
13. **Matsudaira, P.** 1987. Sequence from picomole quantities of proteins electroblotted onto polyvinylidene difluoride membranes. J. Biol. Chem. **262:**10035-10038.
14. **Miller, J.H.** 1972. Experiments in Molecular Genetics p.352-355, Cold Spring Harbor Laboratory.
15. **Moulder, J.W.** 1991.Interaction of *Chlamydiae* and host cells in vitro. Microbiol. Rev. **55:**143-190.
16. **Moulder, J.W., T. P. Hatch, C.C. Kuo, J. Schachter, and J. Storz.** 1984. *Chlamydia*, Vol.1 pp.729-739. In N.R. Krieg and J.G. Holt (ed.), Bergey's Manual of Systematic Bacteriology. The Williams & Wilkins Co., Baltimore.
17. **Oakley, B. R., D. R. Kirsch, and N. R. Morris.** 1980. A simplified ultrasensitive silver stain for detecting proteins in polyacrilamide gels. Anal. Biochem. **105:**361-363.
18. **Pizza, M., M. Bugnoli, R. Manetti, A. Covacci, and R. Rappuoli.** 1990.The subunit S1 is important for Pertussis toxin secretion. J. Biol. Chem. **265:**17759-17763
19. **Ratti, G., A. Moroni, and R. Cevenini.** 1991. Detection of *Chlamydia trachomatis* DNA in patients with non-gonococcal urethritis using the polymerase chain reaction. J. Clin. Pathol. **44:**564-568.
20. **Ricoi, S., R. Cevenini, E. Cosco, M. Comanducci, G. Ratti, and V. Scarlato.** 1993. Transcriptional analysis of the *Chlamydia trachomatis* plasmid pCT identifies temporally regulated transcripts, anti-sense RNA and σ⁷⁰-selected promoters. Mol. Gen. Genet. **237:**318-326.
21. **Ricci S., G. Ratti. and V. Scarlato.** Transcriptional regulation in *Chlamydia trachomatis* pCT plasmid. Gene, in press.
22. **Saiki, A.K., D. H. Gelfand, S. Stoffell, S. J. Scharf, R. Higuchi, G. T. Horne, K. B. Mullis, and H. A. Erlich.** 1988. Primer-directed enzymatic amplification of DNA with a thermostable DNA polymerase. Science **239:**487-491.
23. **Sambrook, J., E. F. Fritsch, and T. Maniatis.** 1989. Molecular Cloning: A Laboratory Manual, second edition. cold Spring Harbor Laboratory. Cold Spring Harbor, N.Y.
24. **Sanchez, J.C., F. Ravier, C. Pasquali, S. Frutiger, N. Paquet, B. Bjellqvist, D.F. Hochstrasser, G.J. Hughes.** 1992. Improving the detection of protein after transfer to polyvinylidene difluoride membranes. Electrophoresis **13:**715-717
25. **Banger, F., S. Nicklen, and A. R. Coulson.** 1977. DNA sequencing with chain terminating inhibitors. Proc. Natl. Acad. Sci. U.S.A. **74:**5463-5467.
26. **Sarimo, S.S., and M.J. Pine.** 1969. Taxonomic comparison of the amino termini of microbial cell protein. J. Bacteriol. **98:**368-374
27. **Sriprakash, K.S., and E. S. MacAvoy.** 1987. Characterization and sequence of a plasmid from the trachoma biovar of *Chlamydia trachomatis*. Plasmid **18:**205-214.
28. **Tam, J.E., C. H. Davis, R. J. Tresher, and P.B. Wyrick.** 1992. Localization of the origin of replication for the 7.5-kb *Chlamydia* plasmid. Plasmid **27:**231-236.
29. **Studier F.W., A.H. Rosenberg, J.J.Dunn, and J.W. Dubendorff.** 1990. Use of T7 RNA polymerase to direct expression of cloned genes. Methods Enzymol. **185:**60-89.
30. **Studier F.W., and B.A. Moffatt.** 1986. Use of bacteriophage T7 RNA polymerase to direct selective high level expression of cloned genes. J. Mol. Biol. **189:**113-130.
31. **Towbin, H., T. Staehelin, and J. Gordon.** 1979. Electrophoretic transfer of proteins from polyacrylamide gels to nitrocellulose sheets: procedure and some applications. Proc. Natl. Acad. Sci. U.S.A. **76:**4650-4655.
32. **Waller, J-P.** 1963. The NH2-terminal residues of the proteins from cell-free extracts of *E.coli*. J.Mol.Biol. **7:**483-496
33. **Wang, S.P., and J.T. Grayston.** 1970. Immunologic relationship between genital TRIC, lymphogranuloma venereum, and related organisms in a new microtiter indirect immunofluorescence test. Am. J. Ophthal. **70:**367-374
34. **Wülfing, C., and A. Plückthun.** 1994. Protein folding in the periplasm of *Escherichia coli*. Mol. Microbiol. **12:**685-692
35. **Tabor S, and Richardson CC** 1985. Proc. Nat. Acad. Sci. USA **262:**1074-1078
36. **Commanducci M, Manetti R, Bini L, Santucci A, Pallini V, Cevenini R, Sueur J-M and Ratti G** 1994. Infect. Immun. **62(12):**5491-5497

## Claims

1. Purified *Chlamydia trachomatis* pgp3 protein, **characterised in that** (i) the protein possesses conformational epitopes present in native pgp3 and (ii) the protein is capable of recognition by antibodies directed against said epitopes in human serum.

2. Purified *Chlamydia trachomatis* pgp3 protein which retains an antigenic structure capable of detecting antibodies directed against pgp3 conformational epitopes.

3. *Chlamydia trachomatis* pgp3 protein according to claim 1 or claim 2, obtainable by a process comprising:
- Transforming an *E.coli* cell with a vector encoding *Chlamydia trachomatis* pgp3 protein;
- culturing the cell under conditions such that said protein is expressed;
- isolating the periplasmic fraction of the resulting cells;
- dialysing said fraction against piperazine-HCl buffer, pH 5.4;
- loading the dialysed solution through an ion-exchange column;
- eluting the column with a NaCl gradient;
- collecting the pgp3-containing fractions; and
- dialysing pgp3 back into a physiological buffer solution.

4. An immunodiagnostic assay comprising at least one step involving as at least one binding partner a protein as defined in any one claims 1 to 3, optionally labelled or coupled to a solid support.

5. The immunodiagnostic assay of claim 5, wherein the assay is ELISA.

6. An immunodiagnostic kit for performing an assay according to claim 4 or claim 5, comprising at least one protein as defined in any one of claims 1 to 3.

7. A method for the production of the protein of any one of claims 1 to 3, comprising culturing a host cell transformed with a vector comprising a polynucleotide encoding a protein according to any one of claims 1 to 3, and isolating the protein.

8. The method of claim 7, wherein the vector comprises the ORF3 gene of any *Chlamydia trachomatis* serotype variant under IPTG-inducible expression control.

9. The method of claim 7 or claim 8, wherein the host cell is *E.coli*.

10. The method of any one of claims 7 to 9, wherein the isolation includes one or more purification steps under non-denaturing conditions.

11. The method of any one of claims 7 to 9, wherein the isolation includes the step of ion-exchange chromatography.

12. The method of any one of claims 7 to 11, wherein the isolation includes the step of preparing a periplasmic extract.

13. A vaccine or therapeutic composition comprising the protein of any one of claims 1 to 3 and a pharmaceutical carrier.

14. The protein of any one of claims 1 to 3 for use in the manufacture of a medicament for vaccinating against *Chlamydia trachomatis* infection or treating such an infection.

## Patentansprüche

1. Aufgereinigtes *Chlamydia trachomatis*-pgp3-Protein, **dadurch gekennzeichnet, dass** (i) das Protein Konformationsepitope besitzt, die im nativen pgp3-Protein vorhanden sind, und (ii) das Protein durch gegen diese Epitope gerichtete Antikörper in menschlichem Serum erkannt werden kann.

2. Aufgereinigtes *Chlamydia trachomatis*-pgp3-Protein, welches eine antigene Struktur beibehält, die den Nachweis durch Antikörper ermöglicht, die gegen pgp3-Konformationsepitope gerichtet sind.

3. *Chlamydia trachomatis*-pgp3-Protein nach Anspruch 1 oder 2, erhältlich durch ein Verfahren umfassend:
- Transformieren einer *E.coli*-Zelle mit einem Vektor, der *Chlamydia trachomatis*-pgp3-Protein codiert;
- Züchten der Zelle unter Bedingungen, unter denen dieses Protein exprimiert wird;
- Isolieren der periplasmatischen Fraktion der resultierenden Zellen;
- Dialysieren der Fraktion gegen Piperazin-HCl-Puffer, pH 5,4;
- Aufbringen der dialysierten Lösung auf eine Ionenaustauschersäule;
- Eluieren der Säule mit einem NaCl-Gradienten;
- Sammeln der pgp3 enthaltenden Fraktionen; und
- Rückdialysieren von pgp3 in eine physiologische Pufferlösung.

4. Immundiagnostischer Test, umfassend mindestens einen Schritt, der als mindestens einen Bindungspartner ein Protein, wie in einem der Ansprüche 1 bis 3 definiert, beinhaltet, gegebenenfalls markiert oder an einen festen Träger gekoppelt.

5. Immundiagnostischer Test nach Anspruch 5, wobei der Test ein ELISA ist.

6. Immundiagnostischer Kit zur Durchführung eines Tests nach Anspruch 4 oder Anspruch 5, umfassend mindestens ein Protein, wie in einem der Ansprüche 1 bis 3 definiert.

7. Verfahren zur Herstellung eines Proteins nach einem der Ansprüche 1 bis 3, umfassend das Züchten einer Wirtszelle, die mit einem Vektor transformiert ist, der ein Polynucleotid umfasst, welches ein Protein nach einem der Ansprüche 1 bis 3 codiert, und das Isolieren des Proteins.

8. Verfahren nach Anspruch 7, wobei der Vektor das ORF3-Gen einer beliebigen *Chlamydia trachomatis*-Serotypenvariante unter IPTG-induzierbarer Expressionskontrolle umfasst.

9. Verfahren nach Anspruch 7 oder 8, wobei die Wirtszelle *E.coli* ist.

10. Verfahren nach einem der Ansprüche 7 bis 9, wobei das Isolieren einen oder mehrere Aufreinigungsschritte unter nicht-denaturierenden Bedingungen beinhaltet.

11. Verfahren nach einem der Ansprüche 7 bis 9, wobei das Isolieren den Schritt einer Ionenaustauschchromatographie beinhaltet.

12. Verfahren nach einem der Ansprüche 7 bis 11, wobei das Isolieren den Schritt der Herstellung eines periplasmatischen Extrakts beinhaltet.

13. Impfstoff oder therapeutische Zusammensetzung, umfassend das Protein nach einem der Ansprüche 1 bis 3 und einen pharmazeutischen Träger.

14. Protein nach einem der Ansprüche 1 bis 3 zur Verwendung in der Herstellung eines Medikaments zur Impfung gegen eine *Chlamydia trachomatis*-Infektion der Behandlung einer solchen Infektion.

## Revendications

1. Protéine pgp3 purifiée de *Chlamydia trachomatis*, **caractérisée en ce que** (i) la protéine possède les épitopes conformationnels présents dans la pgp3 native et (ii) la protéine est capable d'être reconnue par les anticorps dirigés contre lesdits épitopes dans un sérum humain.

2. Protéine pgp3 purifiée de *Chlamydia trachomatis* conservant une structure antigénique capable de détecter des anticorps dirigés contre les épitopes conformationnels de pgp3.

3. Protéine pgp3 de *Chlamydia trachomatis* selon la revendication 1 ou la revendication 2, pouvant être obtenue par un procédé comprenant :
- la transformation d'une cellule d'*E. coli* avec un vecteur codant pour la protéine pgp3 de *Chlamydia trachomatis* ;
- la culture de la cellule dans des conditions telles que ladite protéine soit exprimée ;
- l'isolement de la fraction périplasmique des cellules obtenues ;
- la dialyse de ladite fraction contre un tampon pipérazine-HCl, pH 5,4 ;
- le chargement de la solution dialysée dans une colonne échangeuse d'ions ;
- l'élution de la colonne avec un gradient de NaCl ;
- le recueil des fractions contenant pgp3 ; et
- la dialyse de pgp3 en retour contre une solution physiologique tamponnée.

4. Test de diagnostic immunologique comprenant au moins une étape mettant en jeu, en tant qu'au moins un partenaire de liaison, une protéine selon l'une quelconque des revendications 1 à 3, de façon optionnelle marquée ou couplée à un support solide.

5. Test de diagnostic immunologique selon la revendication 4, ce test étant une méthode ELISA.

6. Kit de diagnostic immunologique pour réaliser un test selon la revendication 4 ou la revendication 5, comprenant au moins une protéine selon l'une quelconque des revendications 1 à 3.

7. Méthode pour la production de la protéine selon l'une quelconque des revendications 1 à 3, comprenant la culture d'une cellule hôte transformée par un vecteur comprenant un polynucléotide codant pour une protéine selon l'une quelconque des revendications 1 à 3, et l'isolement de la protéine.

8. Méthode selon la revendication 7, dans laquelle le vecteur comprend le gène ORF3 d'un variant d'un quelconque sérotype de *Chlamydia trachomatis* sous le contrôle d'une expression inductible par l'IPTG.

9. Méthode selon la revendication 7 ou la revendication 8, dans laquelle la cellule hôte est *E. coli*.

10. Méthode selon l'une quelconque des revendications 7 à 9, dans laquelle l'isolement comprend une ou plusieurs étapes de purification dans des conditions non-dénaturantes.

11. Méthode selon l'une quelconque des revendications 7 à 9, dans laquelle l'isolement comprend l'étape de chromatographie échangeuse d'ions.

12. Méthode selon l'une quelconque des revendications 7 à 11, dans laquelle l'isolement comprend l'étape de préparation d'un extrait périplasmique.

13. Vaccin ou composition thérapeutique comprenant la protéine selon l'une quelconque des revendications 1 à 3, et un véhicule pharmaceutique.

14. Protéine selon l'une quelconque des revendications 1 à 3, pour son utilisation dans la fabrication d'un médicament pour la vaccination contre une infection à *Chlamydia trachomatis* ou pour le traitement d'une telle infection.
